(19) Europäisches Patentamt
European Patent Office
Office européen des brevets

(11) Numéro de publication: **0 376 778**
**A1**

(12) **DEMANDE DE BREVET EUROPEEN**

(21) Numéro de dépôt: **89403400.8**

(51) Int. Cl.5: **C07C 45/46, C07C 49/84**

(22) Date de dépôt: **08.12.89**

(30) Priorité: **14.12.88 FR 8816439**

(43) Date de publication de la demande:
**04.07.90 Bulletin 90/27**

(84) Etats contractants désignés:
**AT BE CH DE ES FR GB GR IT LI LU NL SE**

(71) Demandeur: **RHONE-POULENC CHIMIE**
**25, quai Paul Doumer**
**F-92408 Courbevoie Cédex(FR)**

(72) Inventeur: **Gilbert, Laurent**
**72, rue de Vauban**
**F-69006 Lyon(FR)**
Inventeur: **Rochin, Christophe**
**21, rue Dussaussoy**
**F-69006 Lyon(FR)**

(74) Mandataire: **Ricalens, François et al**
**RHONE-POULENC CHIMIE Service Brevets**
**Chimie 25, quai Paul Doumer**
**F-92408 Coubevoie(FR)**

(54) **Procédé de préparation de cétones aryliques trifluoroéthoxylées.**

(57) La présente invention concerne un procédé de préparation d'arylacétotrifluoroéthoxyphénylcétone selon lequel on condense d'abord le trifluoroéthanol et un iodo ou un bromobenzène dans un solvant aprotique polaire, puis on élimine le dit solvant et on ajoute dans le milieu réactionnel un acide ou un dérivé d'acide arylacétique ainsi que de l'acide fluorhydrique.

EP 0 376 778 A1

Xerox Copy Centre

## PROCEDE DE PREPARATION DE CETONES ARYLIQUES TRIFLUOROETHOXYLEES

La présente invention concerne un procédé de préparation de cétones diaryliques trifluoroéthoxylées. Elle concerne plus particulièrement la préparation d'aryltrifluoroéthoxyacétophénones.

Il est connu de préparer ces cétones portant un groupe trifluoroéthoxy par exemple selon le brevet EP 227055 par condensation sur l'hydroxyphénylacétophénone, soit du paratoluènesulfonate de trifluoroéthyle, soit du méthane sulfonate de trifluoroéthyle, soit du trifluorométhylsulfonate de trifluoroéthyle. Ce brevet décrit aussi la préparation des aryltrifluoroéthylthioacetophénones par condensation du trifluoro éthylthiobenzène avec l'acide ou un dérivé d'acide phényl ou arylacétique en présence d'un acide de Lewis et notamment du trichlorure d'aluminium.

La méthode de préparation des dérivés trifluoroéthoxylés rappellée ci-dessus est difficile à mettre en oeuvre car les sulfonates de trifluoroéthyle ne sont pas disponibles industriellement à un prix intéressant, la méthode décrite pour la préparation des dérivés porteurs du groupe trifluoroéthylthio est difficile à mettre en oeuvre en présence d'un acide de Lewis du type $AlCl_3$, car ce dernier n'est pas recyclable et donc polluant et provoque en plus au cours de la condensation une perte en atomes de fluor.

La présente invention a permis de trouver un procédé industriel c'est-à-dire mettant en oeuvre des matières premières disponibles et peu coûteuses et un catalyseur facilement recyclable et non polluant. Elle permet cependant de ne préparer que les cétones diaryliques portant un groupe trifluoroéthoxylé.

Elle consiste à préparer les diaryl cétones portant un groupe trifluoroéthoxy et notamment les trifluoroéthoxyacétophénones par un procédé en deux étapes sans purification intermédiaire et sans utilisation de catalyseur de Friedel et Crafts.

Elle consiste : - dans une première étape à mettre en contact un iodo ou un bromobenzène avec du trifluoroéthanol en présence d'un sel de cuivre et d'une base forte dans un solvant aprotique polaire,

. dans une deuxième étape à éliminer le solvant aprotique polaire à mettre en contact le milieu obtenu à avec un acide ou un dérivé d'acide aryl acétique au sein de l'acide fluorhydrique liquide en l'absence de catalyseur.

La première étape peut être schématisée par l'équation suivante :

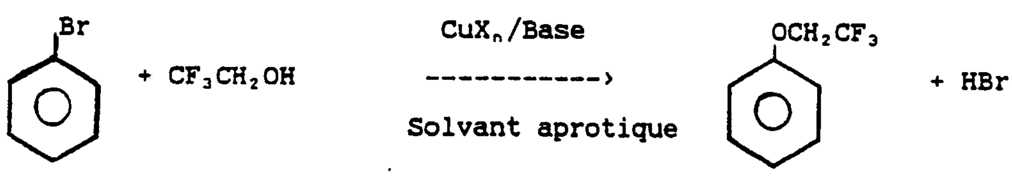


où X = Cl, Br n = 1 ou 2

La deuxième étape peut être schématisée par l'équation suivante :

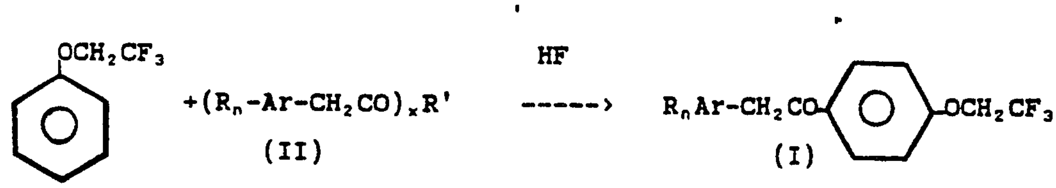


(II) (I)

dans laquelle :

- Ar représente un radical aromatique mono, polycyclique ou hétérocyclique,
- R représente un radical choisi parmi les groupes alkyl, alcoxy, alkylthio, halogénoalkyl, halogénoalcoxy, halogénoalkylthio, amino, nitro, halogéno,
- n est égal à 0, 1, 2 ou 3
- x est égal à 1 ou 2
- R′ représente un radical hydroxy, halogéno quand x = 1 et oxygène quand x est égal à 2.

La première étape est une étape connue telle que par exemple décrite dans le brevet EP 0197869. Elle est réalisée dans un solvant aprotique polaire tel que le diméthylformamide ou la N-méthyl pyrrolidone en présence d'un sel de cuivre par exemple un bromure ou un chlorure. La base utilisée est une base forte choisie parmi les hydroxydes ou carbonates alcalins ou alcalinoterreux, le sodium, l'hydrure de sodium, ou l'amidure de sodium. La réaction est réalisée de préférence entre 80 et 200 °C.

A la fin de cette étape on procède selon une première méthode de mise en oeuvre, après filtration des sels de cuivre, à une distillation du solvant à une température comprise entre 60 et 100 °C, sous une pression comprise entre 10 mm et 35 mm de mercure.

Pour éliminer par exemple le diméthylformamide on peut aussi selon une deuxième méthode de mise en oeuvre laver le milieu réactionnel avec de l'eau contenant environ 5% en poids d'acide chlorhydrique.

En fin de réaction, celle ci étant très souvent incomplète le résidu de distillation ou de lavage contient le trifluoroéthoxybenzène et une quantité résiduelle de bromobenzène. Ces composés sont difficilement séparables car ils ont des points d'ébullition voisins.

Ce mélange est introduit directement dans le réacteur d'acylation de la deuxième étape sans aucune séparation des dérivés obtenus à la première étape. L'acylation réalisée dans l'acide fluorhydrique permet de séparer aisément ces deux composés. En effet, le bromobenzène ne subit aucune acylation alors que le trifluoroéthoxybenzène subit facilement l'acylation dans l'acide fluorhydrique en l'absence de tout catalyseur. La diarylcétone trifluoroéthoxylée est ensuite facilement séparée du milieu réactionnel car elle précipite sous forme de cristaux, le bromobenzène restant en solution.

L'agent d'acylation est représenté par la formule II

$(R_n - Ar - CH_2CO)_xR'$ (II)

dans laquelle Ar, R, n, R′ ont la même signification que précédemment.

On préfère parmi les agents d'acylation de formule II ceux pour lesquels :

- Ar représente un radical benzénique
- R représente un groupe halogéno, perhalogénoalkyl, perhalogénoalcoxy, perhalogénoalkylthio
- n est égal à 1
- x est égal à 1
- R′ représente un radical hydroxy ou chloro

On utilise encore plus préférentiellement les agents d'acylation de formule II dans lesquels R représente le fluor et R′ le chlore.

On peut citer parmi les agents d'acylation :

- l'acide parafluorophénylacétique
- le chlorure de l'acide parafluorophénylacétique
- l'acide parachlorophénylacétique et son chlorure
- l'acide paratrifluorométhylphénylacétique et son chlorure
- l'acide paratrifluorométhoxyphénylacétique et son chlorure

La réaction a lieu dans l'acide fluorhydrique de préférence anhydre.

Pour une meilleure mise en oeuvre de l'invention, on préfère utiliser un rapport molaire de l'acide ou de son dérivé ou trifluoroéthoxybenzène compris entre 1 et 5.

La quantité d'acide fluorhydrique utilisée est telle que le rapport molaire de l'acide fluorhydrique à l'acide de formule (II) ou à son dérivé est compris entre 5 et 50.

La température de réaction est de préférence comprise entre la température ambiante et 120 °C, préférentiellement entre 50 et 80 °C.

On peut citer parmi les produits obtenus par le procédé de l'invention :

- la parafluorophényl para(trifluoroéthoxy 2,2,2) acétophénone
- la parachlorophényl para(trifluoroéthoxy 2,2,2) acétophénone
- la paratrifluorométhylphényl para(trifluoroéthoxy 2,2,2) acétophénone.

Ces composés sont utiles comme intermédiaire de synthèse dans l'industrie pharmaceutique ou phytosanitaire (EP 227055).

L'invention sera plus complètement décrite à l'aide des exemples suivants qui ne doivent pas être considérés comme limitatifs de l'invention.

## EXEMPLE 1

A. Synthèse du (trifluoro 2,2,2 éthoxy) benzène à partir du bromobenzène et de trifluoroéthanol.

Dans un ballon de 100 ml à 4 tubulures, équipé d'une agitation mécanique, d'un réfrigérant ascendant, on charge :

- 20 ml de diméthylformamide
- 12,2 g de trifluoroéthanol (0,122 mol).

La solution est refroidie et on additionne en 15 minutes par petites fractions 4,9 g de NaH en

suspension dans l'huile de vaseline (60 % en masse) (0,122 mol).

Après la fin de l'addition, le mélange est chauffé 2 heures à 35° C. On additionne alors à 25° C, 2,64 g (12 mmol) de bromure cuivrique anhydre.

On coule ensuite en 15 minutes 19,19 g (0,122 mol) de bromobenzène en solution dans 20 ml de diméthylformamide. La masse réactionnelle est chauffée 2 heures 15 à 145° C.

On filtre et on lave le précipité avec 50 ml d'éther.

Le filtrat est versé dans 100 ml d'une solution aqueuse d'acide chlorhydrique à 5 %, qui est elle-même extraite par deux fois 100 ml d'éther. Les phases organiques sont regroupées, lavées 3 fois avec 100 ml d'eau.

Après séchage et évaporation du solvant, on obtient 19,50 g d'un mélange liquide composé de :
- 70 % en poids de (trifluoro 2,2,2 éthoxy) benzène
- 30 % en poids de bromobenzène

B. Synthèse de la parafluorophényl para(trifluoroéthoxy 2,2,2) acétophénone

Dans un réacteur de 50 ml, on charge à 0° C :
- 20 g d'HF
- 5,02 g du mélange obtenu à l'étape A (contenant 3,52 g de trifluoroéthoxybenzène).

On ajoute ensuite 3,45 g de chlorure d'acide para fluorophénylacétique (0,020 mole). On laisse évaporer l'acide chlorhydrique provenant de la transhalogénation. On ferme le réacteur, on chauffe 1 heure à 80° C sous 4 bars de pression autogène. Après refroidissement, le milieu réactionnel est soutiré sur 50 g de glace pilée et filtré. Le précipité obtenu est lavé avec 2 fois 50 ml d'eau glacée et 2 fois avec 50 ml d'éthanol glacé.

On recueille 6,20 g d'un solide beige (RR = 99 %) qui, après analyse par chromatographie en phase liquide et spectrométrie de masse, est identifié comme étant la parafluorophényl para(trifluroéthoxy 2,2,2) acétophénone.

EXEMPLE 2

On reproduit l'exemple 1 en remplaçant les 3,45 g de chlorure de l'acide para fluorophénylacétique par 3,08 g d'acide para fluorophénylacétique.

On obtient 5,72 g d'un solide analysé comme étant la parafluorophényl para(trifluoroéthoxy 2,2,2) acétophénone.

EXEMPLE 3

3A. On reproduit l''exemple 1A en mettant en oeuvre :
- 10,5 g de trifluoroéthanol (0,105 mol)
- 4,2 g de NaH (0,10 mol)
- 19,19 g de bromobenzène (0,122 mol)

Dans les mêmes conditions qu'à l'exemple 1A, on obtient 18,50 g d'un mélange liquide composé de :
- 50 % en poids de (trifluoro 2,2,2 éthoxy)benzène
- 50 % en poids de bromobenzène.

3B. Préparation de la paratrifluorométhylphényl para(trifluoroéthoxy 2,2,2)acétophénone

De la même façon qu'à l'exemple 1B, on charge :
- 20 g d'HF
- 7,04 g du mélange obtenu à l'exemple 3A (contenant 3,52 g de trifluoroéthoxybenzène) (0,02 mole)
- 4,08 g d'acide para trifluorométhylphénylacétique (0,021 mole).

Après 1 heure de chauffage à 80° C, on obtient 5,6 g de para (4 trifluorométhyl phényl) para-(trifluoroéthoxy 2,2,2)acétophénone (rendement = 78 %).

EXEMPLE 4

4A. On reproduit l'exemple 1A en mettant en oeuvre :

- 15,5 g de trifluoroéthanol (0,155 mol)
- 5,5 g de NaH (0,137 mol)
- 19,19 g de bromobenzène (0,122 mol)

Dans les mêmes conditions qu'à l'exemple 1A, on obtient 19,30 g d'un mélange liquide composé de :

- 90 % en poids de (trifluoro 2,2,2 éthoxy)benzène
- 10 % en poids de bromobenzène

4B. On reproduit l'exemple 1B en chargeant :

- 20 g d'HF
- 3,91 g du mélange obtenu à l'exemple 4A (contenant 3,52 g de trifluoroéthoxybenzène)
- 3,4 g d'acide parachlorophénylacétique.

Après 1 heure de chauffage à 80° C, on obtient 6,3 g de para chlorophényl para(trifluoroéthoxy 2,2,2) acétophénone (rendement 96 %).

## Revendications

1. Procédé de préparation d'arylacétotrifluoroéthoxyphénylcétone caractérisé en ce que :

- dans une première étape on met en présence un iodo ou un bromobenzène et du trifluoroéthanol en présence d'un sel de cuivre et d'une base forte dans un solvant aprotique polaire puis,
- dans une deuxième étape on élimine le solvant aprotique polaire et on met en contact le résidu avec un acide ou un dérivé d'acide arylacétique au sein de l'acide fluorhydrique liquide.

2. Procédé selon la revendication 1 caractérisé en ce que dans la deuxième étape l'acide ou le dérivé d'acide arylacétique répond à la formule (II).

$(Rn - Ar - CH_2 - CO)_x R'$ (II)

dans laquelle :

- Ar représente un radical aromatique mono, polycyclique ou hétérocyclique
- R représente un radical choisi parmi les groupes alkyl, alcoxy, alkylthio, halogénoalkyl, halogénoalcoxy, halogéno alkylthio, amino, nitro, halogéno
- n est égal à 0,1, 2 ou 3
- x est égal à 1 ou 2
- R′ représente un radical hydroxy, halogéno, quand x = 1 ou oxygène quand x est égal à 2

3. Procédé selon la revendication 2 caractérisé en ce que

- Ar représente un radical benzénique
- R représente un groupe halogéno, perhalogénoalkyl, perhalogénoalcoxy, perhalogénoalkylthio
- n est égal à 1 et x est égal à 1
- R′ représente un radical hydroxy ou chloro

4. Procédé selon la revendication 3 caractérisé en ce que

. R représente le fluor
. R′ représente le chlore

5. Procédé selon la revendication 1 caractérisé en ce que l'acide fluorhydrique utilisé est anhydre.

6. Procédé selon la revendication 1 caractérisé en ce que le rapport molaire de l'acide ou du dérivé d'acide arylacétique au composé issu de la condensation de la 1ère étape est compris entre 1 et 5.

7. Procédé selon la revendication 1 caractérisé en ce que le rapport molaire de l'acide fluorhydrique à l'acide arylacétique ou au dérivé d'acide arylacétique est compris entre 5 et 50.

8. Procédé selon la revendication 1 caractérisé en ce que la température de réaction de la deuxième étape est comprise entre la température ambiante et 120° C et de préférence entre 50 et 80° C.

Office européen des brevets

# RAPPORT DE RECHERCHE EUROPEENNE

Numero de la demande

EP 89 40 3400

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,Y | EP-A-0 227 055 (SCHERING AG)<br>* Page 5; page 9, ligne 24 *<br>--- | 1 | C 07 C 45/46<br>C 07 C 49/84 |
| Y | FR-A-2 348 181 (BAYER AG)<br>* Page 3, revendication 1 *<br>--- | 1,5,8 | |
| D,X | EP-A-0 197 869 (RHONE-POULENC SPECIALITES CHIMIQUES)<br>* Pages 1,2; revendication 1 *<br>--- | 1 | |
| A | GB-A-2 045 760 (RIKER LABORATORIES INC.)<br>* Exemple 3 *<br>----- | 1 | |

**DOMAINES TECHNIQUES RECHERCHES (Int. Cl.5)**

C 07 C 45/00
C 07 C 49/00

Le présent rapport a été établi pour toutes les revendications

| Lieu de la recherche | Date d'achèvement de la recherche | Examinateur |
|---|---|---|
| LA HAYE | 12-03-1990 | BONNEVALLE E.I.H. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)